# EUROPEAN PATENT APPLICATION

(11) **EP 2 672 253 A1**
(43) Date of publication of application: **11.12.2013**
(21) Application number: 13170776.2
(22) Date of filing: 06.06.2013
(51) Int. Cl.: G01N 21/47, G01N 33/553, G01N 21/65

(54) **Sensor for detection of a target of interest**

(30) Priority: 06.06.2012 US 201261656354 P; 15.03.2013 US 201313835379
(71) Applicant: National Taiwan University, 10617 Taipei (TW)
(72) Inventor: Lin, Shiming, Taipei (TW); Lee, Si-Chen, Taipei 10617 (TW); Chang, Luan-Yin, Taipei (TW)
(74) Representative: Meyer-Dulheuer, Karl-Hermann

(57) **Abstract**

A sensor for detecting a target of interest comprises a light source that emits light, a light receiver for receiving light, a sampling unit disposed between the light source and the ligh receiver for binding the target of interest (901), a light selecting unit for allowing light of a predetermined wavelength be received by the light receiver, and a detector configured to generate an electrical signal, and the magnitude of which reflects the amount of light that is received by the light receiver. The sampling unit comprises a substrate (110), a material (111) disposed on the substrate, and a probe (113) disposed on the material and configured to bind to the target of interest. The sampling unit is configured such that the probe is in the path of the light emitted by the light source so that the light is scattered if the target binds to the probe.

## Description

### BACKGROUND OF THE DISCLOSURE

A biological sensor is an analytical device for detecting a target molecule by interaction of a biomolecule with the target molecule. Compared to culturing or polymerase chain reaction (PCR), a biological sensor can detect the existence of the target molecule within a relatively short time period. Some biological sensors use chromatographic immunoassay techniques. However, these chromatographic immunoassay-based biological sensors have adoption issues. For example, most chromatographic immunoassay-based biological sensors detect antibodies which are generated by the patients after a later stage of infection/disease.

### BRIEF DESCRIPTION OF THE DRAWINGS

Fig. 1 shows an illustrative embodiment of a sensor for detecting a target of interest (e.g., a biomolecule);
Fig. 2A shows an illustrative embodiment of a container of a sensor for detecting a target of interest (e.g., a biomolecule);
Fig. 2B shows another illustrative embodiment of a container of a sensor for detecting a target of interest (e.g., biomolecule);
Fig. 2C shows yet another illustrative embodiment of a container of a sensor for detecting a target of interest (e.g., a biomolecule);
Fig. 3 shows an illustrative embodiment of a sensor for detecting a target of interest (e.g., a biomolecule);
Fig. 4A shows an illustrative embodiment of a sensor for detecting a target of interest (e.g., a biomolecule);
Fig. 4B shows an illustrative embodiment of a sensor for detecting a target of interest (e.g., a biomolecule);
Fig. 4C shows an illustrative embodiment of a sensor for detecting a target of interest (e.g., a biomolecule);
Fig. 4D shows an illustrative embodiment of a sensor for detecting a target of interest (e.g., a biomolecule);
Fig. 5 shows an illustrative embodiment of a method for making a sensor;
Fig. 6A is a chart illustrating the signal strength during detection, by the sensor, of Enterovirus 71 in a 10% diluted sample collected from an infected patient;
Fig. 6B is a chart illustrating the signal strength during detection, by the sensor, of Enterovirus 71 in a control sample collected from a healthy person;
Fig. 7A is a chart illustrating the signal strength during detection, by the sensor, of Influenza A in a 10% diluted sample collected from an infected patient;
Fig. 7B is a chart illustrating the signal strength during detection, by the sensor, of Influenza A in a control sample collected from a healthy person;
Fig. 8A is a chart illustrating the signal strength during detection, by the sensor, of Influenza B in a 10% diluted sample collected from an infected patient; and
Fig. 8B is a chart illustrating the signal strength during detection, by the sensor, of Influenza B in a control sample collected from a healthy person, all arranged in accordance with embodiments of the disclosure.

### SUMMARY

Some embodiments of the present disclosure may generally relate to a sensor for detecting a target of interest. The sensor may comprise a light source that emits light, a light receiver for receiving light, a sampling unit for binding the target of interest disposed between the light source and the light receiver, a light selecting unit for allowing light of a predetermined wavelength be received by the light receiver, and a detector configured to generate an electrical signal. The magnitude of the electrical signal reflects the amount of light that is received by the light receiver. The sampling unit may comprise a substrate, a material disposed on the substrate, and a probe disposed on the material and configured to bind to the target of interest. Furthermore, the sampling unit is configured such that the probe is in the path of the light emitted by the light source.

Some additional embodiments of the present disclosure may generally relate to a sensor for detecting a target of interest that comprises a light source that emits light of a predetermined wavelength, a light receiver for receiving light, a sampling unit for binding the target of interest disposed between the light source and the light receiver, and a detector configured to generate an electrical signal. The magnitude of the electrical signal reflects the amount of light that is received by the light receiver. The sampling unit may comprise a substrate, a material disposed on the substrate, and a probe disposed on the material and configured to bind to the target of interest. The sampling unit is configured such that the probe is in the path of the light emitted by the light source.

The foregoing summary is illustrative only and is not intended to be in any way limiting. In addition to the illustrative aspects, embodiments, and features described above, further aspects, embodiments, and features will become apparent by reference to the drawings and the following detailed description.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here. It will be readily understood that the aspects of the present disclosure, as generally described herein, and illustrated in the figures, can be arranged, substituted, combined, and designed in a wide variety of different configurations, all of which are explicitly contemplated and make part of this disclosure.

In this disclosure, the term "probe" generally refers to a substance (e.g., a biomolecule) that is capable of binding to a target of interest (e.g., a biomolecule). For example, a probe may have a binding affinity for the target of about 100 piconewtons (pN) to about 500 pN. Nonlimiting examples of probes include antibodies, antibody fragments that retain the ability to bind to the target of interest, nucleic acids (e.g., DNA, RNA, aptamers), antigens, and enzymes. In a sensor as described herein, a single probe that recognizes a single target of interest, or two or more probes that recognize a single target or multiple targets of interest may be used.

The term "target" generally refers to any molecule that is detectable with a sensor as described herein. A target may include, but is not limited to, a biomolecule. Examples of targets that are detectable in the sensors described herein include, but are not limited to, biomolecules (for example, virus, proteins, nucleic acids, carbohydrates, lipids), and other types of molecules (e.g., small molecules) such as, haptens, and toxins. In some embodiments, the target is a biomolecule that is present in a bodily fluid and/or tissue.

In some embodiments, a sensor for detecting a target of interest (e.g., a biomolecule) includes a light source, a sampling unit, and a light receiver having a light detector that generates an electrical signal that is proportionate to the amount of light received by the light receiver. The sampling unit may include a container. At least one inner surface of the container includes probes immobilized on a material that is disposed on the container surface. The light source is configured to generate light that passes through the container and eventually received by the light detector. The light may be of a specific wavelength. Depending on the target to be detected, the specific wavelength may be changed accordingly. The specific wavelength may be determined by any technical feasible approaches. In some embodiments, the specific wavelength is determined by scanning the target with the visible light spectrum or UV light spectrum. The maximum absorption wavelength of the target in the visible light spectrum may be the specific wavelength. For example, any of enterovirus 71, influenza A virus, and influenza B virus has a maximum absorption at approximately 560 nanometer (nm) wavelength in the visible light spectrum and has a relatively greater absorption at about 280 nm wavelength in the ultra-violet spectrum. Adenovirus has a maximum absorption at about 340 nm wavelength in the visible light spectrum and has a relatively greater absorption at about 280 nm wavelength in the ultra-violet spectrum. If the target is present and bound to the probes, light is scattered as it passes through the container, amplifying the signal and resulting in a higher level of light reaching the light receiver, and a larger magnitude electrical signal generated by the light detector.

In some embodiments, the light source may generate visible light or ultra-violet light. A light filter may be placed between the light source and the container so that the light entering the container has a specific wavelength. Alternatively, the light filter may be placed between the container and the light detector so that the light entering the light detector has a specific wavelength. Alternatively, some optical elements (e.g., slit, grating, mirror and a linear charge-coupled device) may be placed between the container and the light detector to produce monochromatic light having a specific wavelength from the visible light that passed through the container, and allow the monochromatic light to enter the light detector. Alternatively, the light source may be a monochromatic light source.

In some embodiments, the container includes a substrate, a material disposed on the substrate and one or more probe(s) disposed on the material. The probe is immobilized on the material. The probe may be a biological substance, for example, an antibody, an antibody fragment, a nucleic acid, an aptamer, an antigen or an enzyme, or any substance that is capable of binding to a target of interest in a manner such that light scattering occurs when the target is bound to the probe to a greater degree than when no target is bound. The material is compatible with the probe and the probe can be immobilized on the material. The material may be, for example, a metal such as gold, silver, copper and nickel. The substrate may be composed of any composition that is technically feasible for the material to be disposed thereon, and that does not interfere with detection of a target of interest as described herein. Some examples of suitable substrates may include, without limitation, glass, metal, silicon or polymers.

The material includes a pattern configured to enhance the light scattering when the target is bound to the probe. In some embodiments, the pattern itself is configured to scatter light when the light travels through the pattern. In some embodiments, the pattern may be a film coated on the substrate. In some other embodiments, the coated film may be annealed. The annealing makes the surface of the coated film becomes uneven. The uneven surface may enhance the scattering of the light passing through the container, and may provide an increase in the surface area available for the deposition of the probe.

In some embodiments, the pattern may be a metal rod array disposed on the substrate. The three dimensional property of the metal rod array may also provide an increase in the surface area available for the deposition of the probe. The size of a metal rod in the metal rod array is associated with the specific wavelength set forth above. In some exemplary embodiments, the length of any metal rod is neither a multiple nor a factor of the specific wavelength. The width of any metal rod is neither a multiple nor a factor of the specific wavelength. The distance between two adjacent rods is neither a multiple nor a factor of the specific wavelength.

The probe is disposed or immobilized on the material through one or more chemical bonds (e.g., covalent bond) with the material. The probe may form a "lock and key" relationship with the target to be detected by the sensor. For example, the probe may be DNA, RNA, a protein, an antibody, an antibody fragment, an aptamer, an antigen, or an enzyme. In some embodiments, the probe is an antibody and the target is an antigen to which the antibody binds.

A sample potentially including the target is introduced into the sensor and then flows over the probe. If the target exists in the sample, the amount of photons passing through the container before the sample is introduced into the sensor may be different than the amount of photons passing through the container after the sample is introduced into the sensor because the target coupled with the probe scatters photons. If the target does not exist in the sample, the amount of photons passing through the container remains substantially the same because there is no bound target to scatter photons. In some embodiments, the amount of photons absorbed by the sample is higher in the presence of bound target than in the absence of the target, resulting in a difference in the light signal detected when the target is present and the light signal detected when the target is absent.

In some embodiments, a method for making a sensor is disclosed. The method includes providing a material which includes a pattern configured to scatter light or enhance a light scattering associated with the target of interest, and increase the surface area available for binding with the probes, disposing the material on a substrate and disposing the probes configured to interact with the target of interest on the material.

In some embodiments, the material is a film. The substrate may be cleaned before the material is disposed on the substrate. In some embodiments, before the material is disposed on the substrate, an adhesion layer is disposed on the substrate first. Then the material is disposed on the adhesion layer. The adhesion layer may be chromium.

In some other embodiments, the material is an annealed film which has an uneven surface pattern. The material may be annealed at a temperature from about 300 degrees Celsius (°C) to about 500 °C after the material is disposed on the adhesion layer.

In some other embodiments, the material includes a rod array pattern. A photoresist is coated on the substrate and a photolithography process is performed to form the rod array. The size of each rod in the rod array and the distance between two adjacent rods are associated with at least one of the specific wavelength, the size of the probe, and the size of the target of interest.

In some embodiments, a method for detecting a target of interest with a sensor described herein is disclosed. The sensor includes a light source, a container and a light detector. The container includes a substrate, a material disposed on the substrate and a probe configured to interact with the target of interest. The probe is disposed and immobilized on the material. The method includes transmitting light from the light source through the container and obtaining a first signal based on the light received by a light detector of the sensor.

The method also includes placing a sample that potentially includes the target of interest in the sensor and obtaining a second signal based on the light received by the light detector after the sample is placed in the container. The method further includes comparing the first signal and the second signal and determining whether the target exists in the sample based on the comparison.

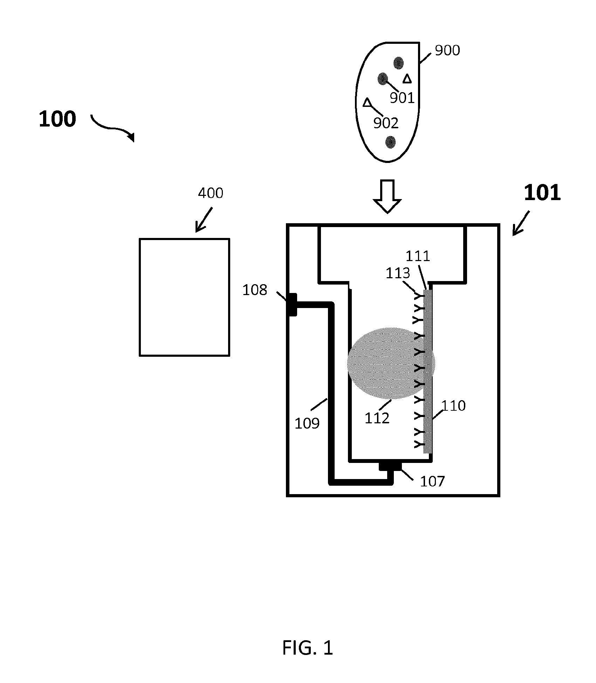

FIG. 1 is an illustrative embodiment of a sensor 100 for detecting a target of interest. The sensor 100 includes a sampling unit. The sampling unit includes a container 101. The container forms an apparatus for binding a target of interest, and includes a material 111 disposed on a substrate 110 (e.g., one or more walls of the container) and probes 113 that are capable of binding to the target disposed on the material. A light 112 is transmitted from a light source of the sensor 100 to a light receiver of the sensor 100 through the container 101. The probes 113 disposed on the material 111 are configured in the path of the light emitted by the light source. The probes 113 are configured such that light passing through the apparatus is scattered when the target of interest is bound to the probes 113.

A solution that does not contain the target, such as a buffer solution, is introduced into the container 101. The light 112 is configured to pass through the container 101 in a first time slot and is received by the light receiver. The light receiver further includes a light detector, such as a photodiode detector, configured to generate a first electronic signal based on the amount of the light 112 that is received by the light receiver in the first time slot. The light receiver further includes a processor for processing the first electronic signal.

A sample 900 potentially including the target of interest 901 is then introduced into the container 101. A predetermined amount of time is allowed to lapse so that if the target of interest 901 exists in the sample 900, the target of interest 901 may bind to the probes 113 on the material 111. After the predetermined period of time has lapsed, introduced sample 900 in the container 101, including impurities 902, is removed from the container 101 by a draining pump 400 through a first hole 107, a passage 109 and a second hole 108, without breaking the bond between the target of interest 901 and the probes 113. Subsequently, the container 101 is rinsed with a buffer solution for a predetermined number of times. For example, fresh buffer solution may be repeatedly injected into and pumped out from the container 101 several times.

Light scattering that occurs when target molecules are bound to the probes 113 on the apparatus will be detected, indicating presence of the target 901 in the sample 900. The light 112 is configured to pass through the container 101 in a second time slot and is received by the light receiver. The photodiode detector of the light receiver is configured to generate a second electronic signal based on the amount of the light that is received by the light receiver in the second time slot. The processor of the light receiver is configured to further process the second electronic signal. If the strength of the second signal differs significantly from the first signal, the processor determines that the target of interest exists in the sample. For example, if the second signal is significantly stronger than the first signal, the processor determines that the target of interest is present in the sample.

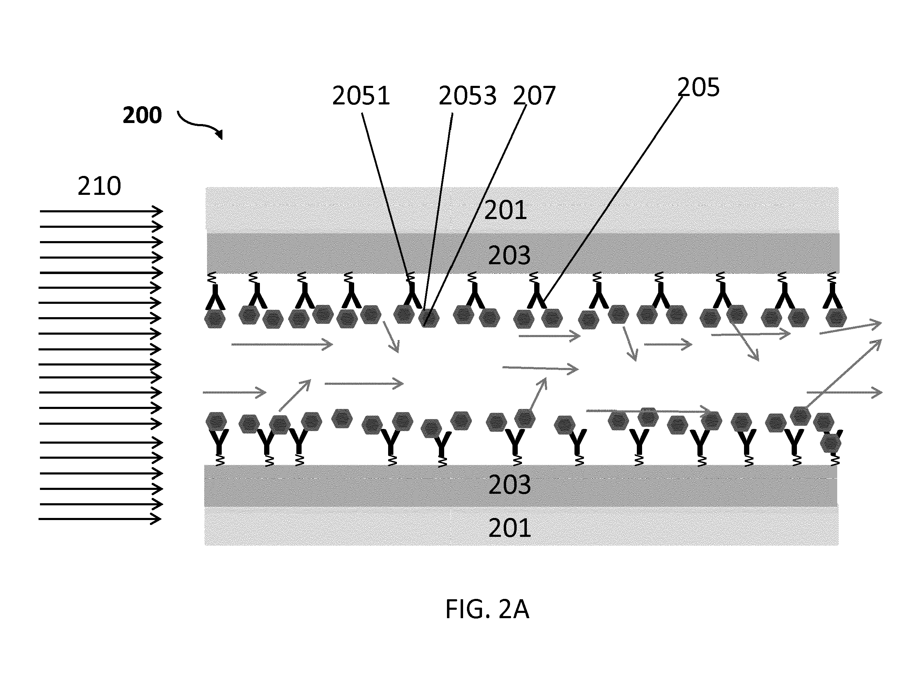

Fig. 2A shows an illustrative embodiment of an apparatus for binding a target of interest. The apparatus 200 includes a substrate 201, a film 203 disposed on the substrate and a probe 205 disposed on the film 203. The thickness of the film 203 may be, for example, a substantially even thickness of about 5nm to about 200nm. A first surface 2051 of the probe 205 is disposed on the material 203. A second surface 2053 of the probe 205 having a binding affinity for the target 207 is configured such that it is available to couple with the target 207 when the target 207 is present. The probes 205 are configured on the film 203 such that light 210 (shown as arrow in Fig. 2A) is scattered when the target 207 binds to the second surface 2053 of probes 205.

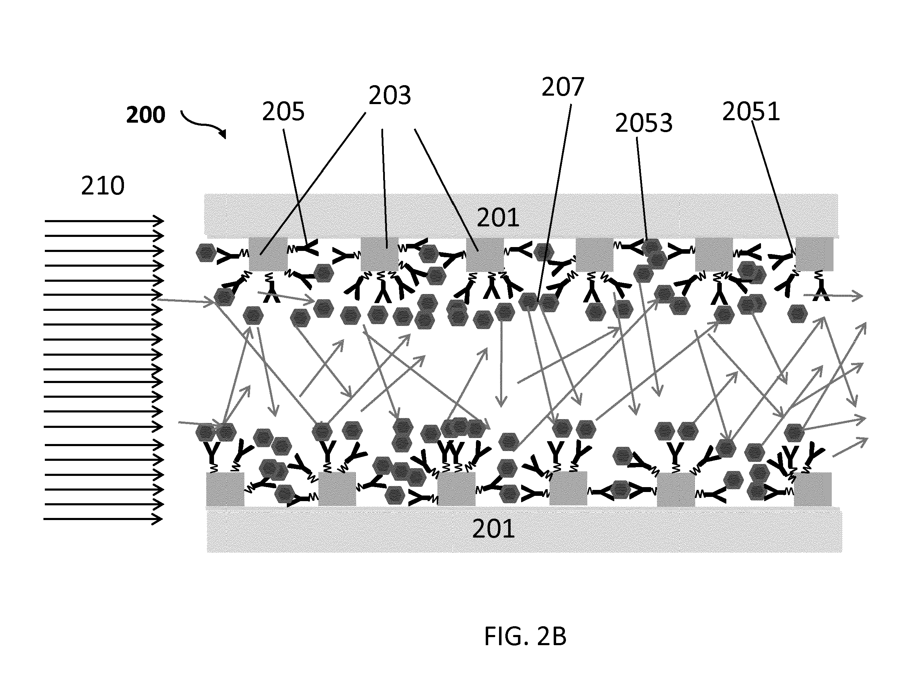

Fig. 2B shows an illustrative embodiment of an apparatus for binding a target of interest. The apparatus 200 includes a substrate 201, a rod array 203 disposed on the substrate and a probe 205 disposed on the rod array 203. The width and the length of any rod in the rod array 203 and the distance between two adjacent rods are associated with at least one of the wavelength of light transmitted from a light source of the sensor, the size of the probe 205 and the target of interest 207. The wavelength is specific to a target of interest 207. The distance between two adjacent rods is also associated with such wavelength. In some embodiments, the width, the length and the distance are all neither a multiple of the wavelength, nor a factor of the wavelength. In some embodiements, the rod of the rod array 203 may have a length from 200 to 900 nm, a width from 200 to 900 nm and a height from 15 to 1500 nm. The distance between each rod in the rod array may be from 200 to 900nm. In some embodiments, the rod of the rod array may have a length of approximately 500 nm, a width of approximately 500 nm and a height of about 100 nm, and the distance between each rod may be approximately 500 nm. A first surface 2051 of the probe 205 is disposed on the rod array 203. A second surface 2053 of the probe 205 having a binding affinity for the target 207 is configured such that it is available to couple with the target 207 when the target 207 is present. The probes 205 are configured on the rod array 203 such that light 210 (shown as arrow in Fig. 2B) is scattered when the target 207 binds to the second surface 2053 of probes 205.

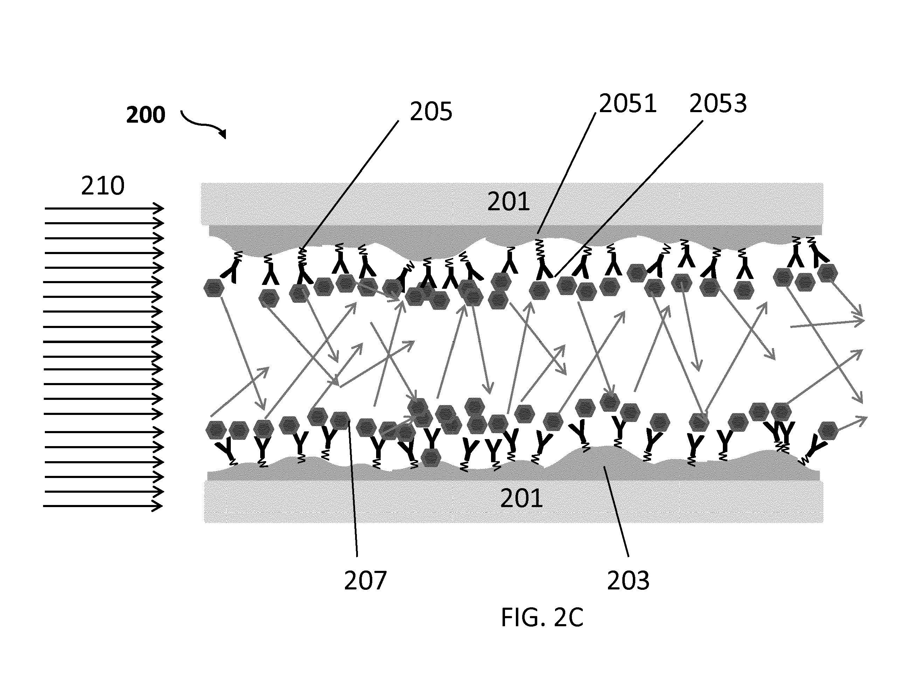

Fig. 2C shows an illustrative embodiment of an apparatus for binding a target of interest. The apparatus 200 includes a substrate 201, a film 203 disposed on the substrate and a probe 205 disposed on the film 203. The film 203 has an uneven thickness. In some embodiments, the film may be first coated on the substrate 201 and then annealed to form the uneven thickness. In some embodiments, the thickness of the film 203 may vary from approximately 0.5nm to approximately 30 nm. A first surface 2051 of the probe 205 is disposed on the film 203. A second surface 2053 of the probe 205 having a binding affinity for the target 207 is configured such that it is available to couple with the target 207 when the target 207 is present. The probes 205 are configured on the material 203 such that light 210 (shown as arrow in Fig. 2C) is scattered when the target 207 binds to the second surface 2053 of probes 205.

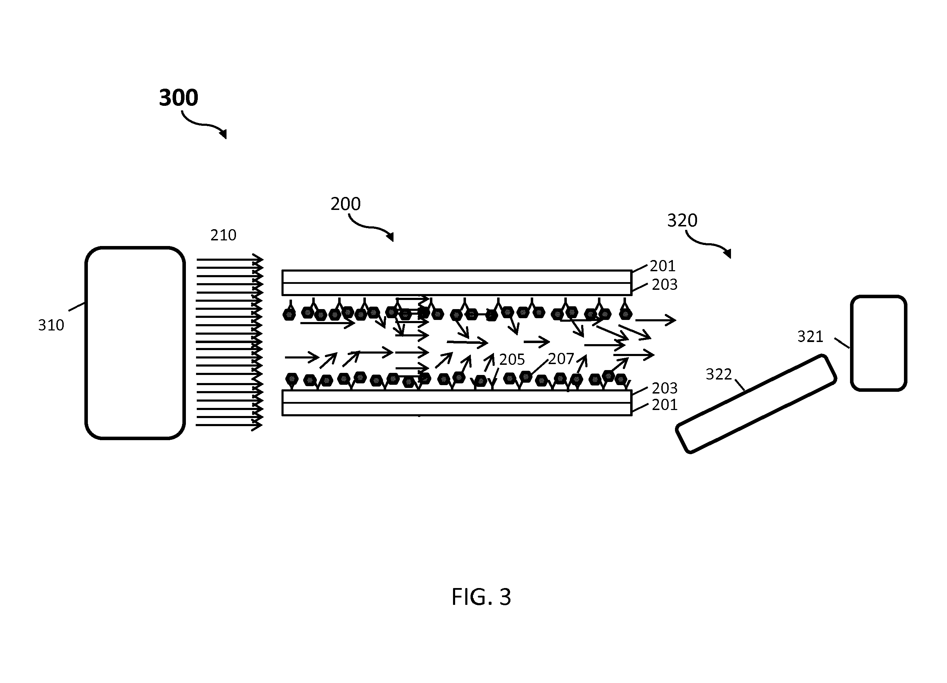

FIG. 3 shows an illustrative embodiment of an apparatus 300 for providing and detecting light passing through an apparatus 200 for detecting a target of interest. The apparatus 300 includes a light source 310 and a light receiving unit 320. The apparatus 200 for detecting a target of interest is disposed between the light source 310 and the light receiving unit 320.

The apparatus 200 for detecting a target of interest may include a substrate 201, a film 203 disposed on the substrate and a probe 205 disposed on the film 203. The configuration of the substrate 201, the film 203 and the probe 205 in the apparatus 200 for detecting a target of interest may be the same or similar to those in the apparatus 200 illustrated and described in reference to FIGs. 2A, 2B and 2C.

Light 210 (shown as arrows in FIG. 3) from the light source 310 passes through the apparatus 200 for detecting a target of interest. When the target 207 binds to the probe 205, the target 207 will absorb some light, and scatter some other light away from the path of the incident light 210. The presence of the target 207 will cause a loss in light power falling on a first photodetector unit 321, which is disposed optically in line with the path of the incident light 210 for transmittance (absorption) detection. Therefore, a first signal may be obtained based on the light received by the first photodetector unit 321 before a sample that potentially includes the target of interest is placed in the apparatus 200 for detecting a target of interest. For instance, the strength of the first signal may be proportional to the intensity of the light received by the first photodetector unit 321. Moreover, a second signal may be obtained based on the light received by the first photodetector unit 321 after the unbound materials in the sample have been removed and replaced with a buffer solution. Furthermore, a processor may compare the difference between the first signal and the second signal, and determine whether or not the target exists in the sample. If the strength of the second signal is significantly less than strength of the first signal, the processor may determine that the target exists in the sample.

In some embodiments, the light receiving unit 320 may further comprise a second photodetector unit 322, which is disposed at an angle relative to the path of the incident light 210 for transmittance (absorption) detection. In some embodiments, the light receiving unit 320 includes the second photodetector unit 322, but not the first photodetector unit 310. The angle may be, for example, any angle that is greater than 0 but less than or equal to 90, allowing the second photodetector unit 322 to receive scattered light. Since the target 207 attached to the probe 205 will scatter the incident light 210, the intensity of the scattered radiation will increase due to the presence of the target 207. Therefore, a first signal may be obtained based on the light received by the second photodetector unit 322 before a sample that potentially includes the target of interest is placed in the apparatus 200 for detecting a target of interest. Moreover, a second signal may be obtained based on the light received by the second photodetector unit 322 after the unbound materials in the sample have been removed and replaced with a buffer solution. Furthermore, a processor may compare the difference between the first signal and the second signal, and determine whether or not the target exists in the sample. If the strength of second signal is significantly stronger than strength of the first signal, the processor may determine that the target exists in the sample.

In some embodiments, the light source may generate visible light or ultra-violet light. A light filter may be placed between the light source and the apparatus 200 for detecting a target of interest so that the light entering the apparatus 200 for detecting a target of interest has a specific wavelength. Alternatively, the light filter may be placed between the=apparatus 200 for detecting a target of interest and the light detector so that the light entering the light detector has a specific wavelength. Alternatively, some optical elements (e.g., slit, grating, mirror and a linear charge-coupled device) may be placed between the apparatus 200 for detecting a target of interest and the light detector to make the visible light (or ultra-violet light) passed through the apparatus 200 for detecting a target of interest turn to a monochromatic light with a specific wavelength before entering the light detector. The light source may also be a monochromatic light source.

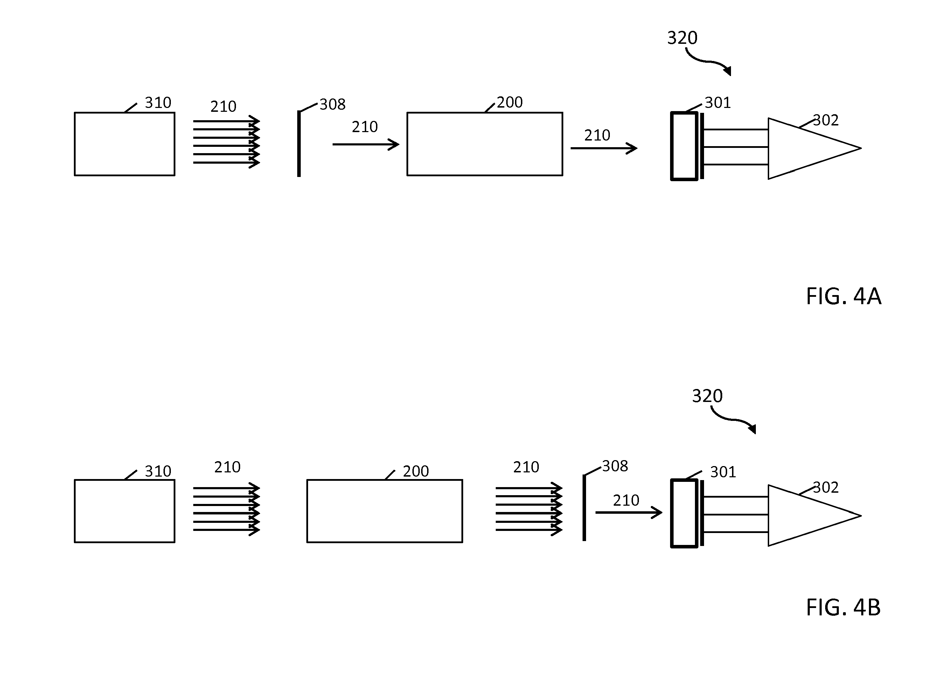

FIG. 4A shows an illustrative embodiment of a sensor for detecting a target of interest (e.g., a biomolecule). For simplicity, a light receiving unit 320 comprising only one photodetector unit is illustrated. One skilled in the art will appreciate that the photodetector unit may be either one of the first photodetector unit 310 and the second photodetector unit 320 illustrated in FIG. 3, and the light receiving unit 320 may comprise additional photodetector units.

The light source 310 in FIG. 4A may emit visible light (shown as arrows in FIG. 4A), and a light filter 308 for selecting a light of a specific wavelength from the visible light may be disposed between the light source 310 and the apparatus 200 for detecting a target of interest. The light may be selected based on the target of interest. For example, if the target of interest is enterovirus 71, influenza A virus or influenza B virus, the light filter 308 may be configured to select a light that has a 560nm wavelength, and if the target of interest is adenovirus, the light filter 308 may be configured to select light that has a 340 nm wavelength.

The light receiving unit 320 may comprise a photodiode chip 301 for receiving the light and a photodetector circuit 302 for measuring the intensity of the selected light that passes through the apparatus 200 for detecting a target of interest and generating an electrical signal that is proportionate to the amount of light received by the light receiving unit 320.

FIG. 4B shows an illustrative embodiment of a sensor for detecting a target of interest (e.g., a biomolecule). The sensor illustrated in FIG. 4B is the same as or similar to the sensor illustrated in FIG. 4A, except the light filter 308 is disposed between the apparatus 200 for detecting a target of interest and the photodiode chip 301, instead of between the light source 310 and the apparatus 200 for detecting a target of interest. Therefore, the light having the wavelength specific to the target is selected after the light has passed through the apparatus 200 for detecting a target of interest.

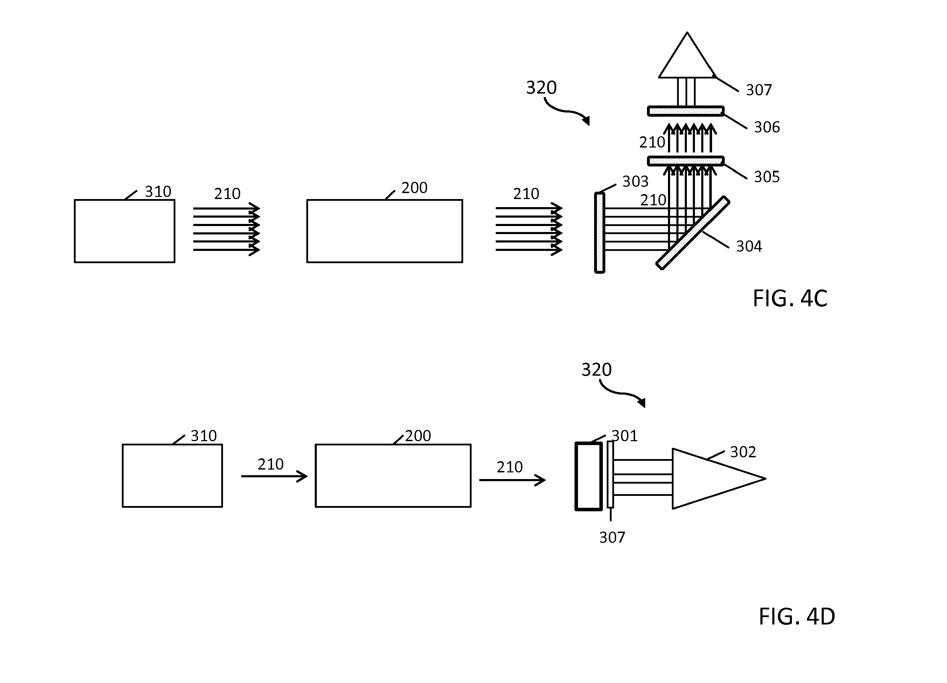

FIG. 4C shows an illustrative embodiment of a sensor for detecting a target of interest (e.g., a biomolecule). The sensor illustrated in FIG. 4C is the same as or similar to the sensor illustrated in FIG. 4A, except that the light filter 308 and photodiode chip 301 are eliminated and the light receiving unit 320 in FIG. 4C comprises a slit 303, a mirror 304, a grating 305, and a linear charge-coupled device (CCD) 306. The visible light that passes through the apparatus 200 for detecting a target of interest enters the slit 303. The grating 305 separates lights of different wavelengths, which are then received by the linear CCD 306, and the photodetector circuit 302 is configured to measure the intensity of the light having the desired wavelength.

FIG. 4D shows an illustrative embodiment of a sensor for detecting a target of interest (e.g., a biomolecule). The sensor illustrated in FIG 4D is the same as the sensor illustrated in FIG. 4A, except that the light source 310 in FIG. 4D emits a monochromatic light having a specific wavelength associated with the target of interest. Therefore, the light filter 308 may be eliminated in the example illustrated in FIG. 4D. The light receiving unit 320 may further comprise an amplifier 307 for amplifying signals received by the photodiode chip 301.

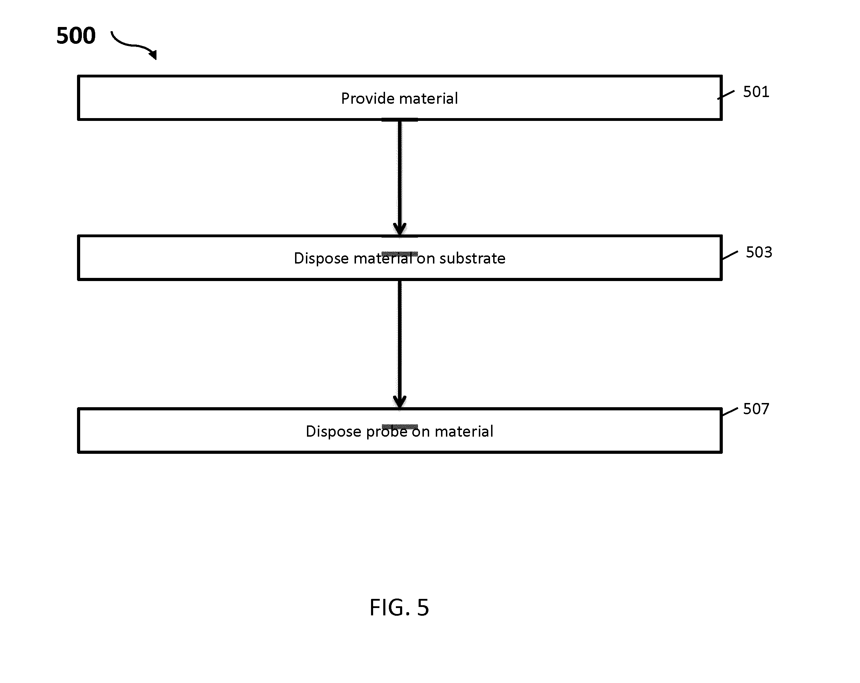

Fig. 5 shows a flow chart of an illustrative embodiment of a method 500 for making an apparatus for binding a target of interest. The method 500 includes steps 501, 503 and 505. In step 501, a material is provided. In step 503, the material is disposed on a substrate. The material may be disposed on the substrate in a pattern configured to increase the surface area available for disposing probes, and to permit and/or enhance scattering light emitted from a light source and traveling across the apparatus when a target of interest is bound to a probe that is disposed on the material. The pattern may be, for example, a film, a film with an uneven thickness, or a rod array. In step 505, a probe that is capable of binding to a target of interest is disposed on the material. The probe is configured to interact with the target that the sensor configured to detect. In some embodiments, before disposing the probe on the material, the material may be cleaned and pre-treated. For example, the material may be cleaned with an acidic solution, a basic solution, and/or purified water. In some embodiments, the material may be pre-treated with one or more compounds. In one embodiment, the material may be pretreated with one or more compound(s) that include(s) at least one functional group compatible with the material. In another embodiment, the material may be pretreated with one or more compound(s) that include(s) at least one functional group compatible with the probe. The functional group is configured to form a first stable bound with the free electrons around the surface of the material and form a second stable bound with the probe. Some example functional groups include, but not limited to, thiol group and hydroxyl group.

### [Example 1]

### [Probe Immobilization]

A glass container configured to contain a sample was placed in a plastic holder and the glass container and the plastic holder were then placed in *pTricorder*® sensor (Vsense Medtech. Co., Ltd., Taipei, Taiwan). Gold was disposed on an inner surface of the container in the form of a film having an uneven thickness from approximately 0.5 nm to approximately 30 nm. Before introducing probes into the container, the gold film was cleaned with a 0.1M hydrochloric acid solution, purified water, 0.1M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Enterovirus 71 monoclonal antibodies was added into the container and incubated for 20 minutes at room temperature to permit anti-Enterovirus 71 monoclonal antibodies to bind to the glutaraldehyde crosslinker. Unbound anti-Enterovirus 71 monoclonal antibody was then removed from the container by a draining pump of the sensor through a hole at the bottom of the glass container. An aqueous solution of 0.5M glycine was then added to the container to react with residual unbound glutaraldehyde. Finally, glycine was removed from and PBS was added to the container.

### [Sample Detection]

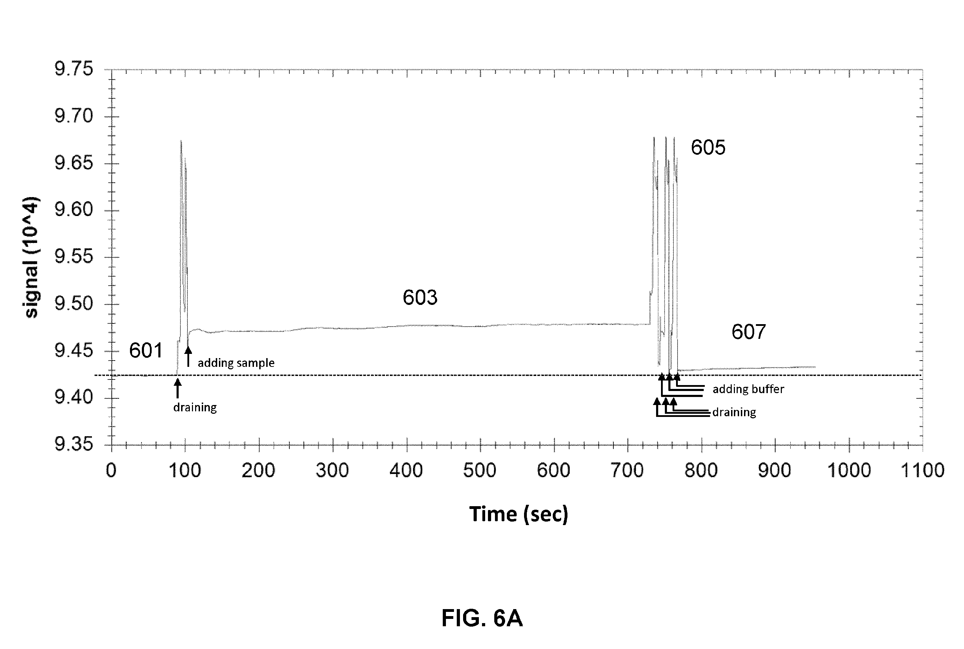

The sensor further includes a visible light source and a light detector for detection of Enterovirus 71. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. FIG. 6A is a chart illustrating the signal strength during detection of Enterovirus 71 in a 10% diluted sample collected from an infected patient. The sample was collected by a throat swab from the infected patient.

The sensor was turned on so that light transmitted from the light source of the sensor passed through the container and over the probes on the inner surface of the container. At this stage, the container contained PBS as set forth above. The signal detected with PBS (as shown at 601 in FIG. 6A) was used as a reference.

After about 1 minute, PBS was removed from the container and the 10% Enterovirus 71 diluted sample was added to the container. Data was collected for 10 minutes. Light detected from the container is shown at 603 in FIG. 6A. After 10 minutes, the Enterovirus 71 diluted sample was removed from the container.

PBS was added to rinse the container to remove nonspecifically bound Enterovirus 71. The rinsing was repeated several times. The rinsing caused various sharp peaks as shown at 605 in FIG. 6A. After rinsing, PBS was added to the container and data was collected for 3 minutes to collect data for light detected from the container (as shown at 607 in FIG. 6A). The difference between the light signal detected at 607 and the signal detected at 601 indicated presence of Enterovirus 71 in the sample.

### [Comparative Example 1]

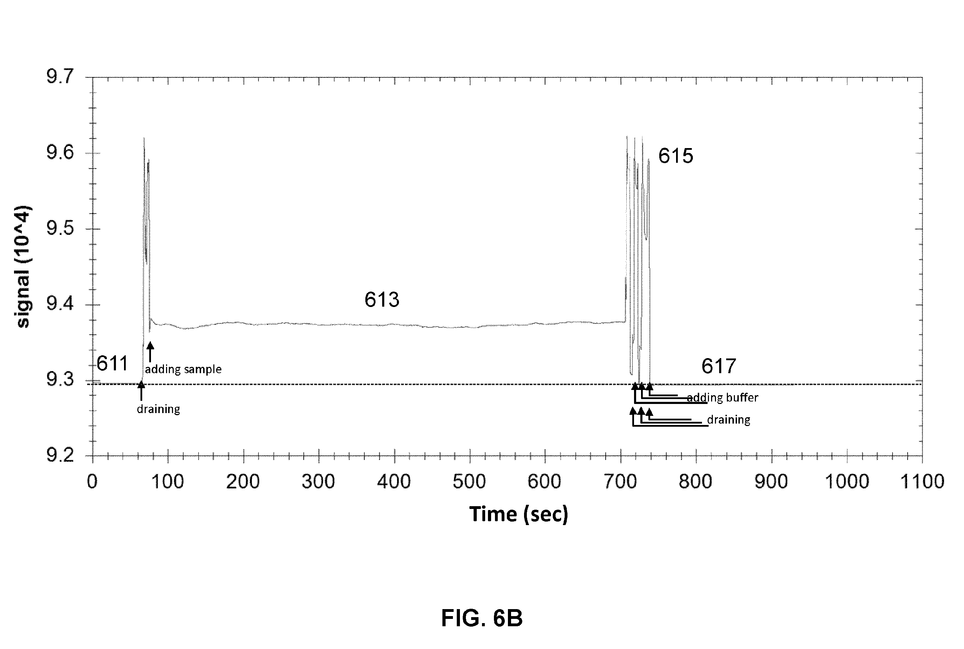

FIG. 6B is a chart illustrating the signal strength during detection of a control sample collected from a healthy person. The detection approach was the same as the approach of the detection of the 10% Enterovirus 71 diluted sample set forth above.

The sensor was turned on so that a light transmitted from a light source of the sensor passed through the container and over the probes on the inner surface of the container. At this stage, the container contained PBS. The signal detected with PBS (as shown at 611 in FIG. 6B) was used as a reference.

After about 1 minute, PBS was removed from the container and the control sample was added to the container. Data was collected for 10 minutes. Light detected from the container is shown at 613 in FIG. 6B. After 10 minutes, the control sample was removed from the container.

PBS was added to rinse the container to remove nonspecifically bound material. The rinsing was repeated several times. The rinsing caused various sharp peaks as shown at 615 in FIG. 6B. After rinsing, PBS was added to the container and data was collected for 3 minutes to collect data for light detected from the container (as shown at 617 in FIG. 6B). The similar signal strength of 611 and 617 showed no existence of Enterovirus 71 in the control sample.

### [Example 2]

### [Probe Immobilization]

A glass container configured to contain a sample was placed in a plastic holder and the glass container and the plastic holder were then placed in *pTricorder*® sensor (Vsense Medtech, Taipei, Taiwan). Gold was disposed on an inner surface of the container in the form of a film having an uneven thickness from about 0.5 nm to about 30 nm. Before introducing probes into the container, the gold film was cleaned with a 0.1M hydrochloric acid solution, purified water, 0.1 M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Influenza A antibody (20µg/ml in PBS solution at pH 7.2) was added into the container and incubated for 20 minutes at room temperature to permit the anti-Influenza A antibody to bind to the glutaraldehyde crosslinker. Unbound anti-Influenza A antibody was then removed from the container by a draining pump of the sensor through a hole at the bottom of the glass container. An aqueous solution of 0.5M glycine was then added to the container to react with residual unbound glutaraldehyde. Finally, glycine was removed from and PBS was added to the container.

### [Sample Detection]

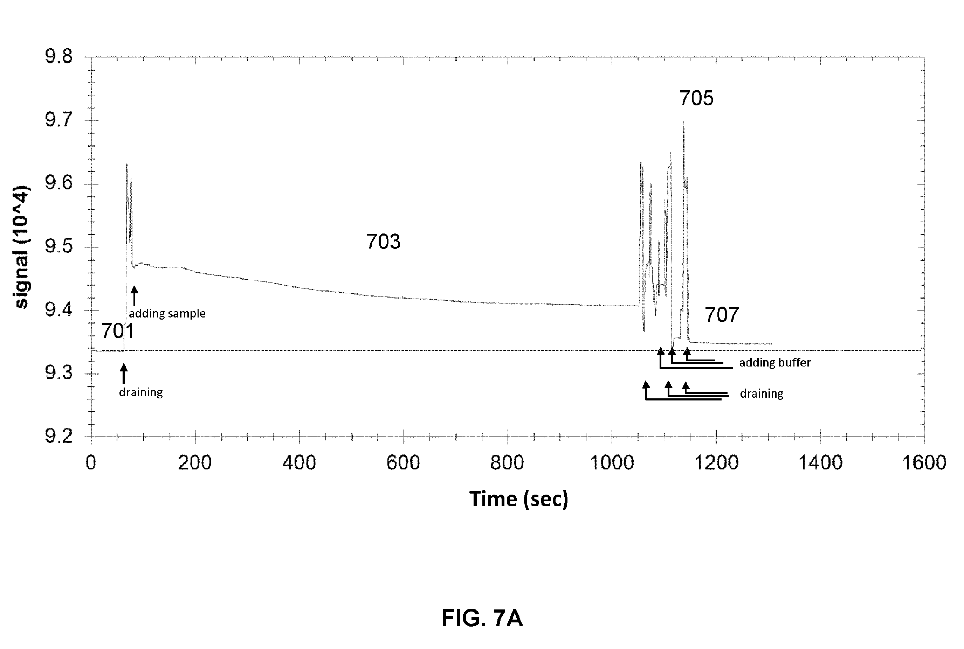

The sensor further includes a visible light source and a light detector for detection of Influenza A. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. FIG. 7A is a chart illustrating the signal strength during detection of Influenza A in a 10% diluted sample collected from an infected patient. The sample was collected by a throat swab from the infected patient's throat.

The sensor was turned on so that light transmitted from a light source of the sensor passed through the container and over the probes on the inner surface of the container. At this stage, the container contained PBS as set forth above. The signal detected with PBS (as shown at 701 in FIG. 7A) was used as a reference.

After about 1 minute, PBS was removed from the container and the 10% Influenza A diluted sample was added to the container. Data was collected for 10 minutes. Light detected from the container is shown at 703 in FIG. 7A. After 10 minutes, the Influenza A diluted sample was removed from the container.

PBS was added to rinse the container to remove nonspecifically bound material. The rinsing was repeated several times. The rinsing caused various sharp peaks as shown at 705 in FIG. 7A. After rinsing, PBS was added to the container and data was collected for 3 minutes to collect data for light detected from the container (as shown at 707 in FIG. 7A). The difference between the light signal detected at 707 and the signal detected at 701 indicated presence of Influenza A in the sample.

### [Comparative Example 2]

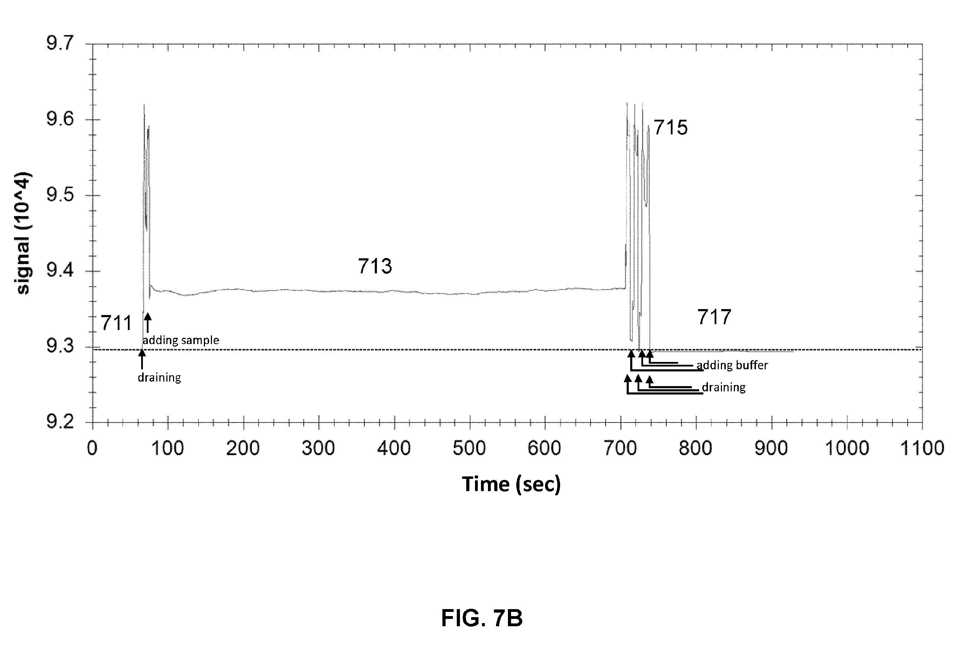

FIG. 7B is a chart illustrating the signal strength during detection of a control sample collected from a healthy person. The detection approach was the same as the approach of the detection of the Influenza A diluted sample set forth above.

The sensor was turned on so that a light transmitted from a light source of the sensor passed through the container and over the probes on the inner surface of the container. At this stage, the container contained PBS. The signal detected with PBS (as shown at 711 in FIG. 7B) was used as a reference.

After about 1 minute, PBS was removed from the container and the control sample was added to the container. Data was collected for 10 minutes. Light detected from the container is shown at 713 in FIG. 7B. After 10 minutes, the control sample was removed from the container.

PBS was added to rinse the container to remove nonspecifically bound material. The rinsing was repeated several times. The rinsing caused various sharp peaks as shown at 715 in FIG. 7B. After rinsing, PBS was added to the container and data was collected for 3 minutes to collect data for light detected from the container (as shown at 717 in FIG. 7B). The similar signal strength of 711 and 717 showed no existence of Influenza A in the control sample.

### [Example 3]

### [Probe Immobilization]

A glass container configured to contain a sample was placed in a plastic holder and the glass container and the plastic holder were then placed in *pTricorder*® sensor (Vsense Medtech Taipei, Taiwan). Gold was disposed on an inner surface of the container in the form of a film having an uneven thickness from about 0.5 nm to about 30 nm. Before introducing probes into the container, the gold film was cleaned with a 0.1M hydrochloric acid solution, purified water, 0.1 M sodium hydroxide, and purified water, in sequence.

After cleaning, an aqueous solution containing 110µL of cystamine (20mM in phosphate buffered saline (PBS) solution at pH 7.2) was added into the container and incubated for 20 minutes at room temperature to permit cystamine to bind to the gold on the container wall. The remaining cystamine solution was then removed from the container. An aqueous solution containing 110µL of glutaraldehyde (2.5% in PBS solution at pH 7.2) was then added into the container and incubated for 20 minutes at room temperature to permit glutaraldehyde to bind to the cystamine.

After removing the remaining glutaraldehyde solution from the container, an aqueous solution of 110µL of commercially available anti-Influenza B antibody (20µg/ml in PBS solution at pH 7.2) was added into the container and incubated for 20 minutes at room temperature to permit the anti-Influenza B antibody to bind to the glutaraldehyde crosslinker. Unbound anti-Influenza B antibody was then removed from the container by a draining pump of the sensor through a hole at the bottom of the glass container. An aqueous solution of 0.5M glycine was then added to the container to react with residual unbound glutaraldehyde. Finally, glycine was removed from and PBS was added to the container.

### [Sample Detection]

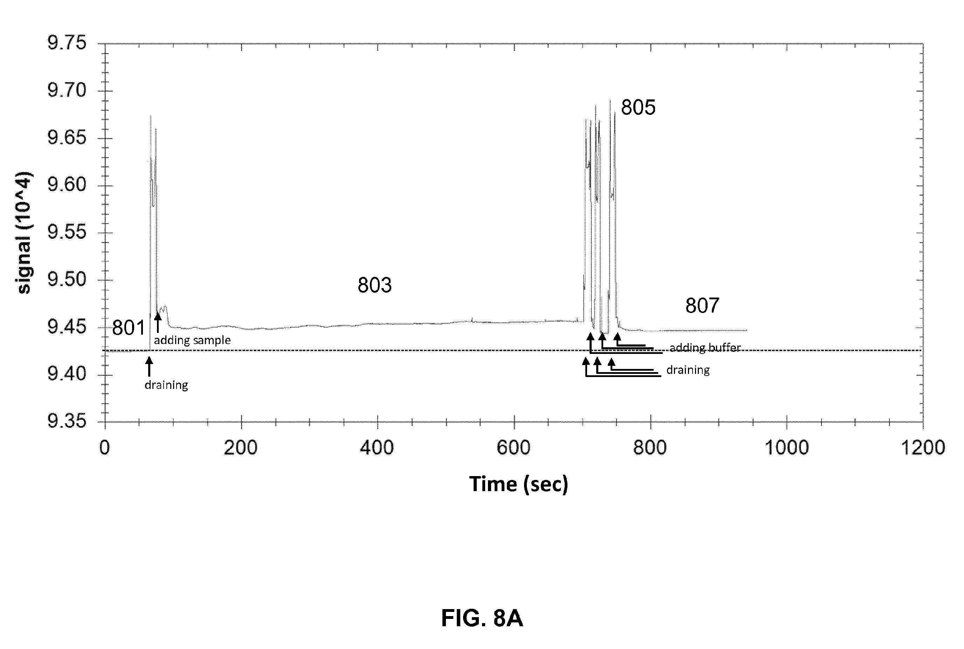

The sensor further includes a visible light source and a light detector for detection of Influenza B. Visible light was transmitted from the visible light source and passed through an optical filter. The optical filter was configured to filter the visible light and only the light having a 560 nm wavelength can pass the optical filter. The light (i.e., having the wavelength of 560 nm) then passed through the glass container set forth above. After passing through the glass container, the light was eventually received by the light detector. FIG. 8A is a chart illustrating the signal strength during detection of Influenza B in a 10% diluted sample collected from an infected patient. The sample was collected by a throat swab from the infected patient's throat.

The sensor was turned on so that light transmitted from a light source of the sensor passed through the container and over the probes on the inner surface of the container. At this stage, the container contained PBS as set forth above. The signal detected with PBS (as shown at 801 in FIG. 8A) was used as a reference.

After about 1 minute, PBS was removed from the container and the 10% Influenza B diluted sample was added to the container. Data was collected for 10 minutes. Light detected from the container is shown at 803 in FIG. 8A. After 10 minutes, the Influenza B diluted sample was removed from the container.

PBS was added to rinse the container to remove nonspecifically bound material. The rinsing was repeated several times. The rinsing caused various sharp peaks as shown at 805 in FIG. 8A. After rinsing, PBS was added to the container and data was collected for 3 minutes to collect data for light detected from the container (as shown at 807 in FIG. 8A). The difference between the light signal detected at 807 and the signal detected at 801 indicated presence of Influenza B in the sample.

### [Comparative Example 3]

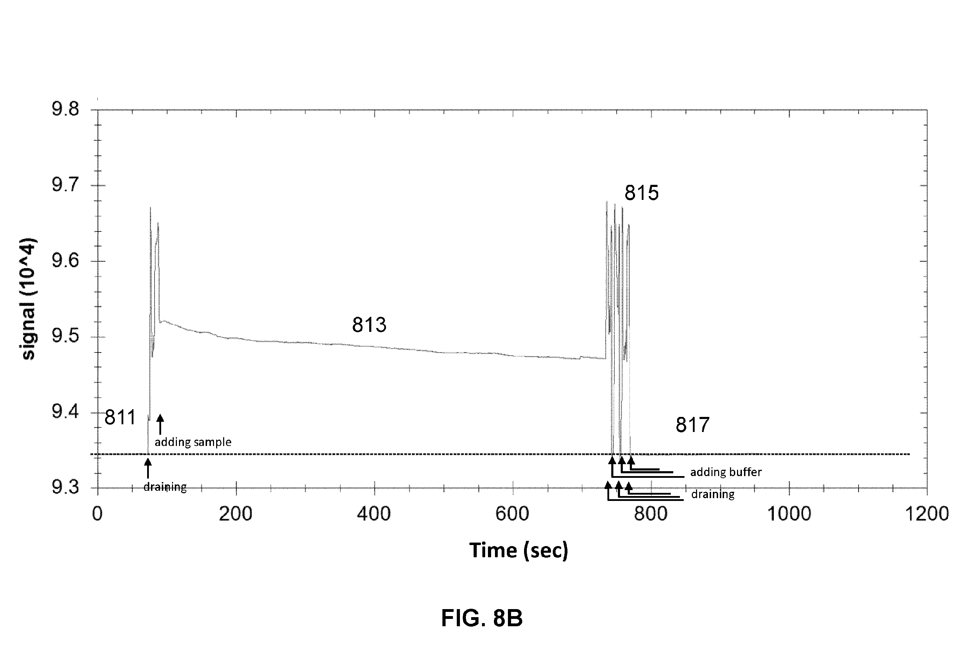

FIG. 8B is a chart illustrating the signal strength during detection of a control sample collected from a healthy person. The detection approach was the same as the approach of the detection of the Influenza B diluted sample set forth above.

The sensor was turned on so that a light transmitted from a light source of the sensor passed through the container and over the probes on the inner surface of the container. At this stage, the container contained PBS. The signal detected with PBS (as shown at 811 in FIG. 8B) was used as a reference.

After about 1 minute, PBS was removed from the container and the control sample was added to the container. Data was collected for 10 minutes. Light detected from the container is shown at 813 in FIG. 8B. After 10 minutes, the control sample was removed from the container.

PBS was added to rinse the container to remove nonspecifically bound material. The rinsing was repeated several times. The rinsing caused various sharp peaks as shown at 815 in FIG. 8B. After rinsing, PBS was added to the container and data was collected for 3 minutes to collect data for light detected from the container (as shown at 817 in FIG. 8B). The similar signal strength of 811 and 817 showed no existence of Influenza B in the control sample.

Although the foregoing invention has been described in some detail by way of illustration and examples for purposes of clarity of understanding, it will be apparent to those skilled in the art that certain changes and modifications may be practiced without departing from the spirit and scope of the invention. Therefore, the description should not be construed as limiting the scope of the invention.

All publications, patents, and patent applications cited herein are hereby incorporated by reference in their entireties for all purposes and to the same extent as if each individual publication, patent, or patent application were specifically and individually indicated to be so incorporated by reference.

## Claims

1. A sensor for detecting a target of interest, comprising:
a light source;
a light receiver;
a sampling unit for binding the target of interest disposed between the light source and the light receiver, the sampling unit comprises:
a substrate;
a material disposed on the substrate; and
a probe disposed on the material and configured to bind to the target of interest, wherein the sampling unit is configured such that the probe is in the path of light emitted by the light source;
a light selecting unit for allowing light of a predetermined wavelength be received by the light receiver; and
a detector configured to generate an electrical signal, the magnitude of which reflects the amount of light that is received by the light receiver.

2. A sensor for detecting a target of interest, comprising:
a light source that emits light of a predetermined wavelength;
a light receiver;
a sampling unit for binding the target of interest disposed between the light source and the light receiver, the sampling unit comprises:
a substrate;
a material disposed on the substrate; and
a probe disposed on the material and configured to bind to the target of interest, wherein the sampling unit is configured such that the probe is in the path of the light emitted by the light source; and
a detector configured to generate an electrical signal, the magnitude of which reflects the amount of light that is received by the light receiver.

3. The sensor of claim 1, wherein the light receiver includes the light selecting unit, which comprises:
a slit for receiving the light, a mirror, a grating, and a linear charge-coupled device ,
wherein the mirror is configured to reflect the received light onto the grating,
the grating is configured to separate the received light into a plurality of lights of different wavelengths and transmit the plurality of lights to the linear charge-coupled device, and
wherein the detector comprises a photodetector circuit for measuring the intensity of one of the plurality of light received by the light receiver and
generating the electrical signal that is proportionate to the intensity of the one of the plurality of light, the one of the plurality of light has the predetermined wavelength.

4. The sensor of claim 1, wherein the light receiver comprises a photodiode chip for receiving the light, and
wherein the detector comprises a photodetector circuit for measuring the intensity of the light received by the light receiver and generating the electrical signal that is proportionate to the intensity of the light received by the light receiver.

5. The sensor of claim 4, wherein the light selecting unit comprises a light filter disposed between the light source and the sampling unit or between the sampling unit and the light receiver.

6. The sensor of one of the preceding claims further comprises an amplifier for amplifying signals received by the light receiver, and/or
wherein the light emitted from the light source comprises a wavelength of about 200 nm to about 800 nm, and/or
wherein the predetermined wavelength is associated with the target of interest.

7. The sensor of one of the preceding claims, wherein the probe comprises DNA, RNA, a protein, an antibody, an antibody fragment, an aptamer, an antigen, or an epitope, and/or
wherein the target of interest is a biomolecule.

8. The sensor of one of the preceding claims, wherein the target comprises a virus, a protein, a nucleic acid, a carbohydrate, a lipid, a hapten, or a toxin, and/or
wherein the probe binds to the target of interest with an affinity of about 100 piconewtons to about 500 piconewtons.

9. The sensor of one of the preceding claims, wherein the material comprises a metal, and/or
wherein the material comprises a pattern configured to scatter light emitted from the light source.

10. The sensor of claim 9, wherein the pattern provides an increase in the surface area available for the deposition of the probe.

11. The sensor of claim 9 or 10, wherein the pattern is further configured to facilitate the probe to scatter light emitted from the light source when the target of interest is bound to the probe.

12. The sensor of claim 9, 10 or 11, wherein the pattern comprises a rod array disposed on the substrate, in particular a rod of the rod array has a length from 200 to 900 nm, a width from 200 to 900 nm and a height from 15 to 1500 nm.

13. The sensor of claim 9, 10 or 11, wherein the pattern comprises a film with a substantially even thickness or an uneven thickness, in particular a substantially even thickness is in the range of 5 nm to 200 nm or an uneven thickness varies from 0.5 nm to 30 nm.

14. The sensor of one of the preceding claims, wherein the light transmitted from the light source is scattered by the probe and the target of interest when the target is bound to the probe.

15. The sensor of one of the preceding claims, wherein the sampling unit is configured for scattering of the light transmitted from the light source when the target of interest is bound to the probe, wherein said light is scattered in accordance with Rayleigh scattering, Mie scattering, Brillouin scattering, Raman scattering, inelastic X-ray scattering or Compton scattering.
